# EUROPEAN PATENT APPLICATION

(11) **EP 4 725 602 A1**
(43) Date of publication of application: **15.04.2026**
(21) Application number: 25204284.1
(22) Date of filing: 24.09.2025
(51) Int. Cl.: B01L 3/00

(54) **CHIP AND ENCAPSULATION METHOD THEREFOR**

(30) Priority: 09.10.2024 CN 202411406306
(71) Applicant: GeneMind Biosciences Company Limited, Shenzhen City, Guangdong 518023 (CN)
(72) Inventor: Xu, Liang, Shenzhen City, 518023 (CN); Shu, Anxin, Shenzhen City, 518023 (CN)
(74) Representative: Michalski Hüttermann & Partner Patentanwälte mbB

(57) **Abstract**

Disclosed in the present invention are a chip and an encapsulation method therefor. The chip comprises a first sheet material, a second sheet material, a first encapsulant and a second encapsulant, the second sheet material being disposed opposite the first sheet material, the first encapsulant being disposed between the first sheet material and the second sheet material, a lane being formed in the first encapsulant, and the second encapsulant being disposed around an edge of the lane. In this way, the first encapsulant can form a stable lane for a reagent to perform a biochemical reaction, and the second encapsulant can isolate the first encapsulant from the lane, reducing or preventing the influence of special molecules released by the first encapsulant on the biochemical reaction in the lanes, thereby improving sequencing quality.

## Description

### TECHNICAL FIELD

The present invention relates to the field of gene sequencing, and in particular to a chip and an encapsulation method therefor.

### BACKGROUND ART

A chip adapted to a sequencing platform is a reaction apparatus that can carry a nucleic acid to be detected and can accommodate a solution to provide a reaction environment or a detection environment for the nucleic acid to be detected; it is also referred to as a flow chamber or a flow cell.

On a platform of an optical system that implements sequencing on the basis of a detection chip (sometimes simply referred to as a sequencer), a specific position of the chip (a position where a nucleic acid molecule to be detected is connected, sometimes also referred to as a reaction region or a fluid lane) is imaged, and then the base sequence of the nucleic acid molecule to be detected is identified and determined on the basis of the information of these images. For example, specifically, in a platform in which nucleotides with optical labels are used to perform sequencing on the basis of the sequencing-by-synthesis principle, during sequencing, a sequencer irradiates labels in a reagent solution and excites them to emit optical signals. Then, the optical signals are collected, for example, by taking pictures, to obtain images. The base sequence is identified and determined on the basis of the information of these images, to achieve the purpose of sequencing.

In the related art, chips include a substrate and a cover plate, the substrate and the cover plate being encapsulated by means of a glue material. Lanes formed between the substrate and the cover plate are unstable, and glue material release is a relatively large problem, which affects a biochemical reaction.

### SUMMARY OF THE INVENTION

The present invention provides a chip and an encapsulation method therefor.

The present application provides a chip. The chip comprises a first sheet material, a second sheet material, a first encapsulant and a second encapsulant, the second sheet material being disposed opposite the first sheet material, the first encapsulant being disposed between the first sheet material and the second sheet material, a lane being hollowed out in the first encapsulant, and the second encapsulant being disposed around a side wall edge of the lane.

In this way, the first encapsulant can form a stable lane for a reagent to perform a biochemical reaction, and the second encapsulant can isolate the first encapsulant from the lane, reducing or preventing the influence of special molecules released by the first encapsulant on the biochemical reaction in the lanes, thereby improving sequencing quality.

In some embodiments, an outer edge of the first encapsulant is flush with outer edges of the first sheet material and the second sheet material.

In some embodiments, the second encapsulant defines the edge of the lane.

In some embodiments, the first encapsulant and the second encapsulant do not overlap in a thickness direction of the first sheet material.

In some embodiments, the second encapsulant connects the first sheet material to the second sheet material.

In some embodiments, the first encapsulant is a pressure-sensitive adhesive.

In some embodiments, the second encapsulant is a UV adhesive.

In some embodiments, a thickness of the first sheet material is less than a thickness of the second sheet material.

In some embodiments, surfaces of the first sheet material and the second sheet material facing each other are both flat surfaces.

In some embodiments a distance between the first sheet material and the second sheet material ranges from 50 µm to 200 µm.

In some embodiments, a width of the second encapsulant ranges from 0.2 mm to 0.3 mm.

In some embodiments, a width of the lane ranges from 3 mm to 15 mm, and/or a length of the lane ranges from 50 mm to 200 mm.

In some embodiments, end portions of the lane in a length direction form a converging shape.

In some embodiments, a minimum distance between two adjacent lanes is 2.0 mm.

The present application provides an encapsulation method for a chip, the method comprising:
disposing a first encapsulant on a first sheet material, a lane being formed in the first encapsulant;
disposing a second encapsulant at an edge of the lane; and
bonding the first sheet material provided with the first encapsulant and the second encapsulant to a second sheet material.

In some embodiments, before disposing the first encapsulant on the first sheet material, the method comprises:
cleaning the first sheet material and the second sheet material; and
drying the cleaned first sheet material and second sheet material.

In some embodiments, disposing the first encapsulant on the first sheet material comprises:
placing the first sheet material on a carrying platform;
placing the first encapsulant on a conveying apparatus;
driving the carrying platform and the conveying apparatus to move relative to each other, so that the carrying platform and the conveying apparatus approach each other, so as to drive the first encapsulant and the first sheet material to approach each other;
using a positioning apparatus to assist in positioning the first sheet material and the first encapsulant; and
controlling a pressing apparatus to bond the first encapsulant to the first sheet material.

In some embodiments, before disposing the second encapsulant on the edge of the lane, the method comprises:
performing defoaming treatment on the first sheet material provided with the first encapsulant by using a defoaming device.

In some embodiments, disposing the second encapsulant on the edge of the lane comprises:
placing the first sheet material on a dispensing jig;
performing positioning by using the first encapsulant; and
dispensing the second encapsulant to the edge of the lane.

In some embodiments, bonding the first sheet material provided with the first encapsulant and the second encapsulant to the second sheet material comprises:
placing the first sheet material provided with the first encapsulant and the second encapsulant and the second sheet material on a bonding jig; and
bonding the first sheet material to the second sheet material by using a vacuum bonding machine.

In some embodiments, after the first sheet material provided with the first encapsulant and the second encapsulant is bonded to the second sheet material, the method comprises:
by using a pressure-holding device, performing pressure holding on the chip after the first sheet material and the second sheet material are bonded together;
curing the chip after pressure holding by using a curing device; and
performing defoaming treatment on the cured chip by using a defoaming device.

In some embodiments, curing the chip after pressure holding by using the curing device comprises:
emitting ultraviolet light to the second encapsulant by using the curing device, so as to cure the second encapsulant.

Additional aspects and advantages of the present invention will be provided in part in the following description, and will become apparent in part from the following description, or may be understood by means of putting the present invention into practice.

### BRIEF DESCRIPTION OF THE DRAWINGS

The above and/or additional aspects and advantages of the present invention will become apparent and readily appreciated from the following description of the embodiments with reference to the drawings, in which:
FIG. 1 is a schematic structural diagram of a chip according to an embodiment of the present invention;
FIG. 2 is a schematic cross-sectional view of FIG. 1 along the direction A-A;
FIG. 3 is a performance comparison diagram of chips packaged with different types of adhesive materials;
FIG. 4 is a flowchart of a chip encapsulation method according to an embodiment of the present invention;
FIG. 5 is a flowchart of a chip encapsulation method according to an embodiment of the present invention;
FIG. 6 is a flowchart of a chip encapsulation method according to an embodiment of the present invention;
FIG. 7 is a flowchart of a chip encapsulation method according to an embodiment of the present invention;
FIG. 8 is a flowchart of a chip encapsulation method according to an embodiment of the present invention; and
FIG. 9 is a flowchart of a chip encapsulation method according to an embodiment of the present invention.

Description of reference signs: 100. Chip; 10. First sheet material; 20. Second sheet material; 21. Inlet; 22. Outlet; 30. First encapsulant; 31. Lane; 32. Middle section; 33. First end; 34. Second end; 40. Second encapsulant.

### DESCRIPTION OF THE PREFERRED EMBODIMENTS

Embodiments of the present invention will be described in detail below, and examples of the embodiments are shown in the drawings, wherein identical or similar reference numerals represent identical or similar elements or elements having identical or similar functions throughout. The embodiments described below with reference to the drawings are exemplary, are only used to explain the present invention, and should not be construed as limiting the present invention.

In the description of the present invention, it should be understood that the orientation or positional relationships indicated by the terms "center," "longitudinal," "lateral," "length," "width," "thickness," "upper," "lower," "front," "rear," "left," "right," "vertical," "horizontal," "top," "bottom," "inner," "outer," "clockwise," "counterclockwise," etc. are based on the orientation or positional relationship shown in the drawings, and are only for the convenience of describing the present invention and simplifying the description, rather than indicating or implying that the apparatus or element referred to must have a specific orientation, or be constructed and operated in a specific orientation, and thus they cannot be construed as limiting the present invention. In addition, the terms "first" and "second" are used for descriptive purposes only, and are not to be construed as indicating or implying relative importance or implicitly indicating the number of technical features indicated. Thus, features defined by "first" and "second" may explicitly or implicitly include one or more of the features. In the description of the present invention, the meaning of "a plurality" is two or more, unless otherwise specifically defined.

In the description of the present invention, it should be noted that, unless otherwise specified and defined, the terms "mounting," "connected" and "connection" should be construed broadly. For example, "connection" may be a fixed connection, a detachable connection, or an integral connection; a mechanical connection or an electrical connection or mutual communication; or a direct connection, an indirect connection through an intermediate medium, or an internal connection between two elements, or an interaction relationship between two elements. For those of ordinary skill in the art, the specific meanings of the above terms in the present invention can be understood on a case-by-case basis.

In the present invention, unless otherwise specified and defined, a first feature being "above" or "below" a second feature may include that the first and second features are in direct contact, or may include that the first and second features are not in direct contact but are in contact through another feature between them. Moreover, a first feature being "above", "over" and "on top of" a second feature includes that the first feature is directly above and obliquely above the second feature, or simply means that the first feature is higher in level than the second feature. A first feature being "below", "under" and "beneath" a second feature includes that the first feature is directly below and obliquely below the second feature, or simply means that the first feature is lower in level than the second feature.

The following disclosure provides many different embodiments or examples to implement different structures of the present invention. In order to simplify the disclosure of the present invention, the components and configurations of specific examples will be described below. Of course, these are only examples and are not intended to limit the present invention. In addition, in the present invention, reference numerals and/or reference letters may be repeated in different examples; such repetition is for the purpose of simplicity and clarity, and does not in itself indicate the relationship between various embodiments and/or arrangements discussed. In addition, the present invention provides examples of various specific processes and materials, but those of ordinary skill in the art may be aware of the application of other processes and/or the use of other materials.

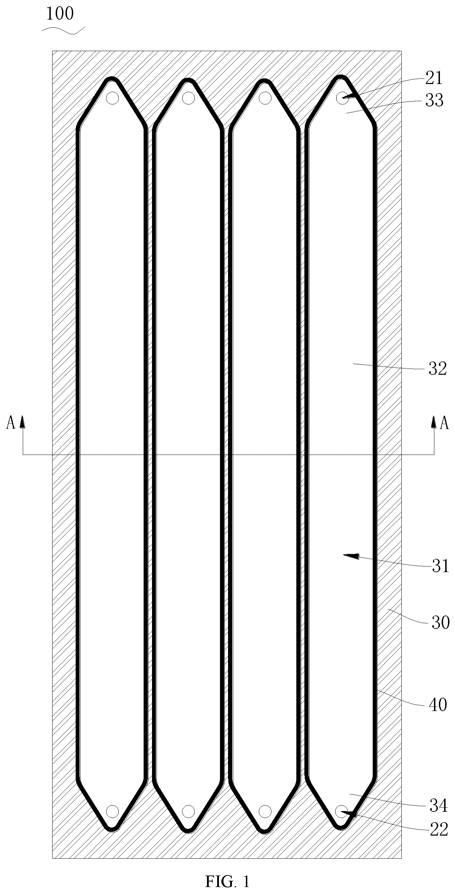

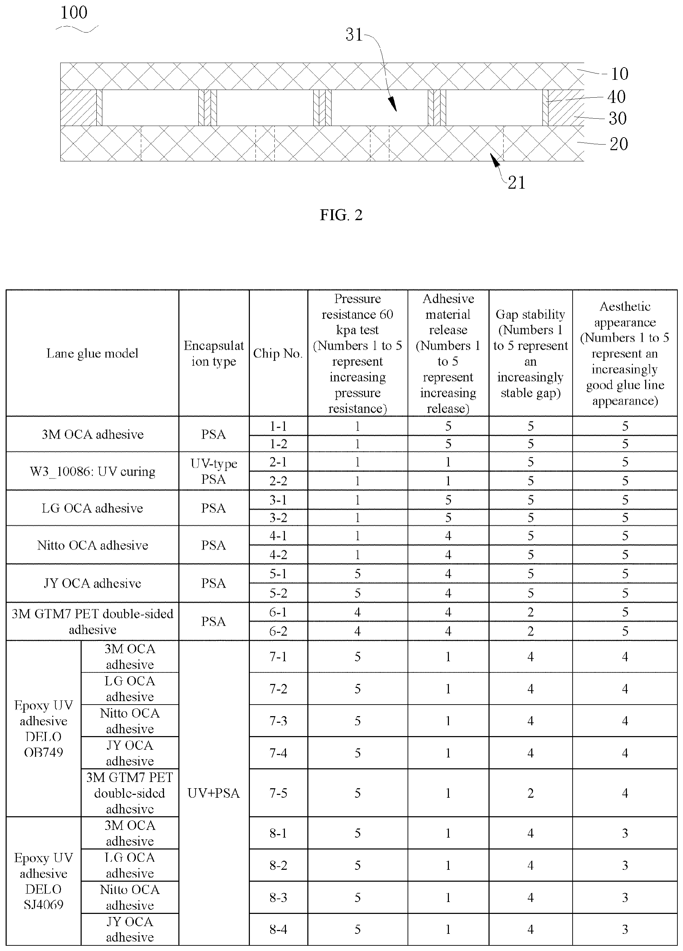

With reference to FIGS. 1 and 2, the present application provides a chip 100. The chip 100 includes a first sheet material 10, a second sheet material 20, a first encapsulant 30, and a second encapsulant 40. The second sheet material 20 is disposed opposite the first sheet material 10, the first encapsulant 30 is disposed between the first sheet material 10 and the second sheet material 20, a lane 31 is hollowed out in the first encapsulant 30, and the second encapsulant 40 is disposed around a side wall edge of the lane 31.

In this way, the first encapsulant 30 can form a stable lane 31 for a reagent to perform a biochemical reaction, and the second encapsulant 40 can isolate the first encapsulant 30 from the lane 31, reducing or preventing the influence of special molecules released by the first encapsulant 30 on the biochemical reaction in the lane 31, thereby improving sequencing quality.

Specifically, the chip 100 may be a reactor on which nucleic acid molecules to be detected with optical detection labels are affixed, and having a space for accommodating liquid, which can be used to affix samples to be detected. The chip is also referred to as a flow chamber or a flow cell.

The first sheet material 10 and the second sheet material 20 may be made of glass, silica, crystal, quartz glass, plastic, ceramic, polyethylene terephthalate (PET), polymethyl methacrylate (PMMA), etc. In one example, the first sheet material 10 and the second sheet material 20 are optically transparent, such as glass sheets/glass layers, so that an optical system can collect an optical signal generated on the surface of the chip 100. The thickness directions of the first sheet material 10, the second sheet material 20, and the first encapsulant 30 may be the same direction.

The first encapsulant 30 is disposed between the first sheet material 10 and the second sheet material 20, and one or more lanes 31, for example, 1, 2, 3, 4, 6, 8, etc., may be hollowed out between the first sheet material 10 and the second sheet material 20 by means of a partially hollow design. The lane 31 has a space for accommodating liquid, and can accommodate a sample to be detected and a reagent, so that the sample to be detected and the reagent perform a biochemical reaction. The arrangement of the lane 31 in the chip 100 may be adjusted on the basis of different requirements. In one embodiment, a lane 31 may extend along the length direction of the first encapsulant 30, and a plurality of lanes 31 may be arranged at intervals along the width direction of the first encapsulant 30. In another embodiment, the lane 31 may be configured as a U-shaped lane having openings facing the same end.

The second encapsulant 40 is disposed around the edge of the lane 31, that is, the shape of the second encapsulant 40 may be consistent with the shape formed by the edge of the lane 31, so as to define the edge of the lane 31 by means of the second encapsulant 40. For example, if the lane 31 is rectangular, the second encapsulant 40 is disposed in a rectangular ring shape.

With reference to FIG. 2, in some embodiments, an outer edge of the first encapsulant 30 is flush with outer edges of the first sheet material 10 and the second sheet material 20; this may be understood as: the first sheet material 10 and the second sheet material 20 are bonded by means of the first encapsulant 30. A lane 31 is formed by means of performing hollowing out processing on the first encapsulant 30.

In this way, the bonding area between the first encapsulant 30 and the first sheet material 10 and second sheet material 20 can be increased, thereby improving the stability of encapsulation of the chip 100.

Specifically, the first encapsulant 30, the first sheet material 10, and the second sheet material 20 have the same shape and size. For example, each of the first encapsulant 30, the first sheet material 10 and the second sheet material 20 is rectangular and has four right-angle corners, and the first encapsulant 30, the first sheet material 10 and the second sheet material 20 have the same length and width. For another example, each of the first encapsulant 30, the first sheet material 10 and the second sheet material 20 is rectangular and has four rounded or chamfered corners, the first encapsulant 30, the first sheet material 10 and the second sheet material 20 have the same length and width, and the rounded corners have the same radius, or the chamfered corners have the same shape and size.

With reference to FIG. 1, in some embodiments, the second encapsulant 40 is in contact with the edge of the lane 31; that is, the second encapsulant defines the edge of the lane.

The second encapsulant 40 may be disposed inside the lane 31 and around the side wall edge of the lane 31, which can prevent a gap from being formed between the second encapsulant 40 and the inner edge of the first encapsulant 30, reducing the overflow of the first encapsulant 30 along the gap direction, and thereby enabling the lane 31 to have a stable depth.

With reference to FIG. 2, in some embodiments, the first encapsulant 30 and the second encapsulant 40 do not overlap in the thickness direction of the first sheet material 10.

This allows the first encapsulant 30 to form a lane 31 having a stable depth, thereby preventing a depth change of the lane 31 from affecting the imaging of the optical system.

Specifically, the first encapsulant 30 connects the first sheet material 10 to the second sheet material 20, the second encapsulant 40 is disposed around a side wall of the lane 31, and an outer edge of the second encapsulant 40 is in contact with an inner edge of the first encapsulant 30.

With reference to FIG. 2, in some embodiments, the second encapsulant 40 connects the first sheet material 10 to the second sheet material 20.

In this way, the second encapsulant 40 can isolate the inner edge of the first encapsulant 30 from the inner side of the lane 31, so as to prevent a gap from being formed between the second encapsulant 40 and the first sheet material 10 or the second sheet material 20, and prevent special molecules released by the first encapsulant 30 from passing through the gap and affecting a biochemical reaction in the lane 31, thereby improving sequencing quality. Specifically, the thickness of the second encapsulant 40 may be the distance between the first sheet material 10 and the second sheet material 20.

In some embodiments, the first encapsulant 30 is a pressure-sensitive adhesive.

The pressure-sensitive adhesive may form a stable lane 31. Specifically, the material body of the pressure-sensitive adhesive may be a resin pressure-sensitive adhesive, resin pressure-sensitive adhesives including polyacrylate, polyurethane, polyvinyl chloride, polyvinyl ether, etc.; or the material body of the pressure-sensitive adhesive may be a rubber pressure-sensitive adhesive, including styrene-butadiene rubber, polyisoprene rubber, polyisobutylene, butyl rubber, chloroprene rubber, nitrile rubber, etc.

Methods of processing and cutting the pressure-sensitive adhesive having a lane 31 include laser cutting, blade die punching, etc. Laser cutting and blade die punching methods have high processing accuracy and high efficiency. Both sides of the processed pressure-sensitive adhesive are protected by release films, and no adhesion effect is generated between the release films and the pressure-sensitive adhesive. That is, in an unused state, the pressure-sensitive adhesive is not damaged by the outside, and in the use process, the protective release films on both sides of the pressure-sensitive adhesive are sequentially removed according to specific needs, an object to be adhered is bonded to the pressure-sensitive adhesive, and the adhered object is firmly adhered by the pressure-sensitive adhesive under the action of external force.

In some embodiments, the second encapsulant 40 is a UV adhesive.

UV adhesives having strong adhesion and stable properties, and do not release special molecules that affect the biochemical reaction of the reagent in the lane 31.

Specifically, the UV adhesive may be an epoxy UV adhesive, an acrylic UV adhesive, a silica gel UV adhesive, or the like. The UV adhesive needs to be applied by corresponding equipment by means of dispensing, spin coating, spraying, etc. Then, an object to be bonded is bonded and affixed, and the UV adhesive is irradiated with ultraviolet light to cure and fix the UV adhesive, thereby achieving the purpose of bonding and fixing.

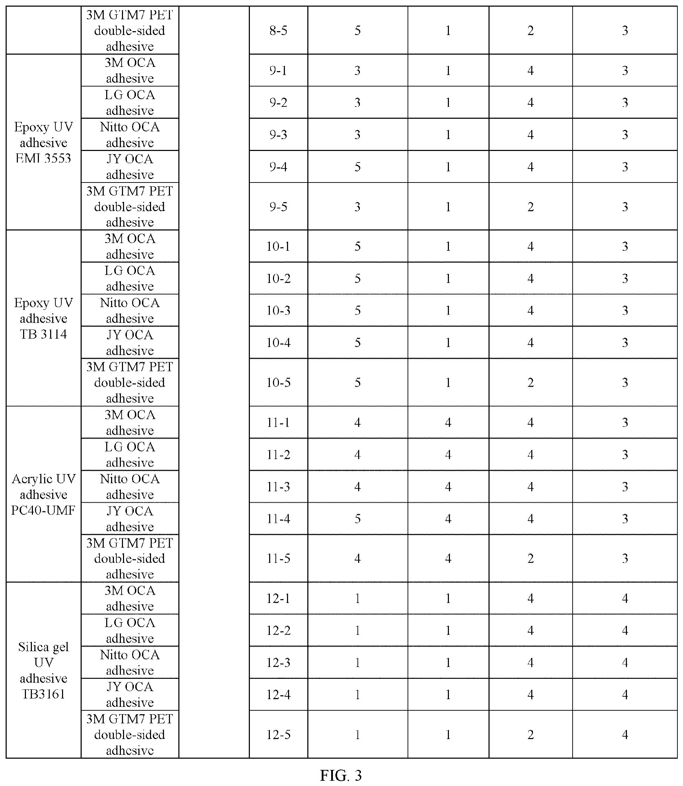

In FIG. 3, chip numbers 1 to 6 of the chip 100 are a comparison of the encapsulation effects of the first sheet material 10 and the second sheet material 20 using different types of pressure-sensitive adhesives, and chip numbers 7 to 12 of the chip 100 are a comparison of the encapsulation effects of the first sheet material 10 and the second sheet material 20 using different types of pressure-sensitive adhesives and UV adhesives. As can be seen from the figure, when the first encapsulation adhesive 30 is an OCA adhesive and the second encapsulation adhesive 40 is an epoxy UV adhesive DELO OB749, the chip 100 has a good pressure resistance effect, low adhesive material release, good stability of the lane 31, and good appearance of the adhesive line.

With reference to FIG. 2, in some embodiments, the thickness of the first sheet material 10 is less than the thickness of the second sheet material 20.

Specifically, the first sheet material 10 may be a cover plate of the chip 100, the second sheet material 20 may be a substrate of the chip 100, and the cover plate may provide a protective effect for the substrate. Moreover, after an analysis process is completed, a sample located in the lane 31 may leak, and the sample can be prevented from overflowing from the chip 100 by means of the function of the cover plate.

The first sheet material 10 may be made of a transparent material, which can ensure that a clear photo can be obtained smoothly when an imaging mechanism takes a photo of the sample, and the photo will not be blurred due to the first sheet material 10. Illustratively, the thickness of the first sheet material 10 ranges from 0.1 mm to 0.5 mm, and the thickness of the second sheet material 20 ranges from 0.5 mm to 1 mm.

In order to form a lane 31 between the first sheet material 10 and the second sheet material 20 of the chip 100 so that fluid can flow therethrough, the second sheet material 20 may be provided with an inlet 21 and an outlet 22, the inlet 21 and the outlet 22 being in communication with the lane 31 so that a biochemical reaction sequencing reagent can flow into the lane 31 from the inlet 21 and flow out from the outlet 22, forming a smooth flow lane.

In some embodiments, the surfaces of the first sheet material 10 and the second sheet material 20 facing each other are both flat surfaces.

In the theoretical simulation calculation process of simulating fluid inside the chip 100, it is found that the flow of the fluid includes conditions such as laminar flow, turbulent flow, and turbulent flow, and influence factors of the uniformity of the flow and distribution of the fluid are not limited to the distribution of flow velocity and the flow line inside the flow channel, but also include the distribution of pressure inside the lane 31, and the distribution of stress inside the lane 31 and the first sheet material 10. Therefore, to ensure the uniformity of the fluid flow and distribution, the height of the lane 31 is the same as the height of the first encapsulant 30, so that the surfaces of the first sheet material 10 and the second sheet material 20 that are disposed opposite to each other have a planar structure, which is convenient for processing and advantageous for reducing costs. Moreover, the planar structure is advantageous for reducing the resistance of the flow of a biochemical reactant in the lane 31, and ensuring uniform pressure on the inner surfaces of the first sheet material 10 and the second sheet material 20 that are disposed opposite to each other, thereby helping to improve the accuracy of gene sequencing.

In some embodiments, the distance between the first sheet material 10 and the second sheet material 20 ranges from 50 µm to 200µm.

Specifically, the distance between the first sheet material 10 and the second sheet material 20 is the thickness of the first encapsulant 30, i.e., the depth of the lane 31. When the depth of the lane 31 is within the described range, it is convenient for a biochemical reaction to occur in the lane 31, and it is also advantageous for double-sided imaging.

The distance between the first sheet material 10 and the second sheet material 20 may be 70 µm, 72 µm, 74 µm, 76 µm, 78 µm, 80 µmetc. The first encapsulant 30 may be selected according to actual needs to have different thicknesses, so as to form lanes 31 having different depths to meet different sequencing requirements. The distance between the first sheet material 10 and the second sheet material 20 may be equal everywhere and within the above range, or may be within the above range everywhere.

In some embodiments, the width of the second encapsulant 40 ranges from 0.2 mm to 0.3 mm.

In this way, when the width of the second encapsulant 40 is within the above range, the first encapsulant 30 can be isolated from the lane 31, and the width of the lane 31 enclosed by the second encapsulant 40 is convenient for biochemical reaction.

Specifically, the width of the second encapsulant 40 is the distance between the inner edge and the outer edge of the second encapsulant 40, i.e., the distance between the inner edge of the second encapsulant 40 and the edge of the lane 31. The width of the second encapsulant 40 may be 0.2 mm, 0.22 mm, 0.24 mm, 0.26 mm, 0.28 mm, 0.3 mm, etc. The amount of the second encapsulant 40 may be set according to the width, thickness and length of the second encapsulant 40, and the length of the second encapsulant 40 is the perimeter of a central ring line.

In some embodiments, the width of the lane 31 ranges from 3 mm to 15 mm, and/or the length of the lane 31 ranges from 50 mm to 200 mm.

In this way, when the width and/or length of the lane 31 is within the described range, a space having a suitable size can be provided for the biochemical reaction, which facilitates the biochemical reaction between the sample to be detected and the reagent in the lane 31.

Specifically, the width of the lane 31 is the maximum dimension of the lane 31 in a width direction, the width direction of the lane 31 being a direction in which a plurality of lanes 31 are arranged at intervals, and the width of the lane 31 may be 5 mm, 6 mm, 7 mm, 8 mm, 9 mm, 10 mm, 11 mm, 12 mm, etc.

The length of the lane 31 is the maximum dimension of the lane 31 in a length direction, the length direction of the lane 31 being perpendicular to the width direction and the thickness direction of the lane 31, and the length of the lane 31 may be 70 mm, 80 mm, 90 mm, 100 mm, 110 mm, 120 mm, 130 mm, etc.

It is possible that the width of the lane 31 ranges from 5 mm to 12 mm, it is also possible that the length of the lane 31 ranges from 70 mm to 130 mm, or it is also possible that the width of the lane 31 ranges from 5 mm to 12 mm and the length of the lane 31 ranges from 70 mm to 130 mm.

With reference to FIG. 1, in some embodiments, end portions of the lane 31 in the length direction form a converging shape.

In this way, it is convenient for the reagent solution to flow into the lane 31 from the end portions, and it is also convenient for the reagent solution in the lanes 31 to gather toward the end portions.

Specifically, the shape of the lane 31 may be irregular. For example, the lane 31 may include a middle section 32, a first end 33, and a second end 34. The first end 33 and the second end 34 are symmetrically disposed at two ends of the lane 31, respectively, and both have a triangular shape. The middle section 32 is in the shape of a long narrow rectangle. Certainly, the first end 33 and the second end 34 may also have different shapes, for example, the first end 33 forming an angled corner, and the second end 34 forming a rounded corner.

The first end 33, the middle section 32 and the second end 34 are sequentially arranged along the length direction of the first encapsulant 30. The middle section 32 is used to allow the reagent solution to react correspondingly, the first end 33 may be configured as a liquid inlet region for allowing the reagent solution to flow into the lane 31, and the second end 34 may be configured as a liquid outlet region for discharging the reagent solution from the lane 31. Certainly, it should be understood that the positions of the first end 33 and the second end 34 may be interchanged, and therefore, the lane 31 may also be configured such that the second end 34 is a liquid inlet region for allowing the reagent solution to flow into the lane 31, and the first end 33 is a liquid outlet region for discharging the reagent solution from the lane 31.

In some embodiments, the minimum distance between two adjacent lanes 31 is 2.0 mm.

In this way, it is possible to reduce the risk of cross-contamination of different samples to be detected and different reagents in different lanes 31 while improving the strength of the first encapsulant 30.

Specifically, the distance between two adjacent lanes 31 may be the distance between the middle sections 32 of the two adjacent lanes 31, and the distance between the two adjacent lanes 31 may be 2.0 mm, 2.2 mm, 2.4 mm, 2.6 mm, 2.8 mm, 3.0 mm, etc.

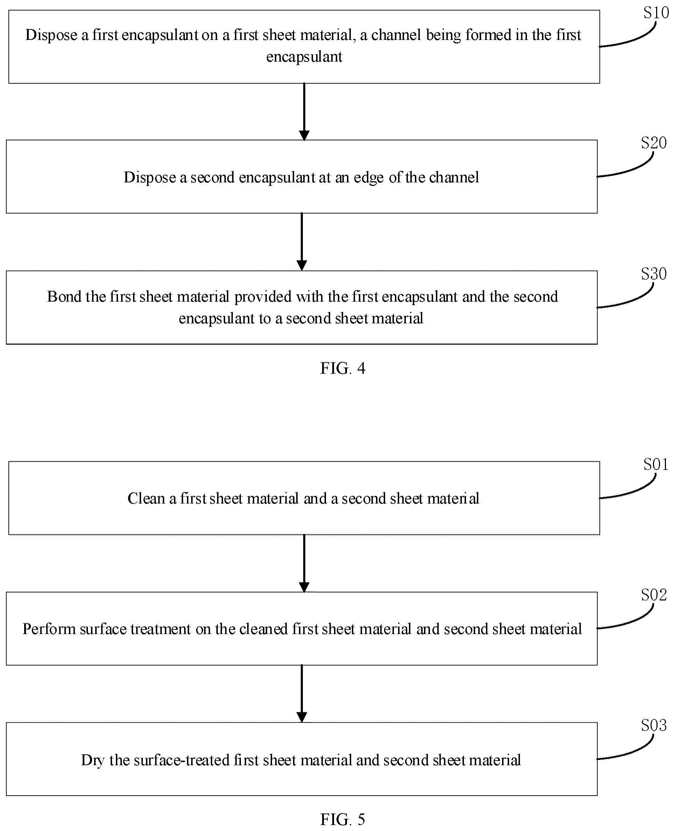

With reference to FIG. 4, the present application provides an encapsulation method for a chip 100, the method comprising:
S10. Disposing a first encapsulant 30 on a first sheet material 10, a lane 31 being formed in the first encapsulant 30;
S20. Disposing a second encapsulant 40 at an edge of the lane 31; and
S30. Bonding the first sheet material 10 provided with the first encapsulant 30 and the second encapsulant 40 to a second sheet material 20.

Specifically, in step S10, before the first encapsulant 30 is disposed on the first sheet material 10, the first encapsulant 30 may be cut, so that a lane 31 is formed in the first encapsulant 30.

In step S20, the second encapsulant 40 may be disposed at the edge of the lane 31 by using a dispensing machine, and the dispensing machine may be a manual dispensing machine, a semiautomatic dispensing machine, or a fully automatic dispensing machine.

In step S30, the first sheet material 10 and the second sheet material 20 may be bonded by using pressure bonding or vacuum bonding.

With reference to FIG. 5, in some embodiments, before the first encapsulant 30 is disposed on the first sheet material 10, the method includes:
S01. Cleaning the first sheet material 10 and the second sheet material 20;
S02. Performing surface treatment on the first sheet material 10 and the second sheet material 20; and
S03. Drying the surface-treated first sheet material 10 and second sheet material 20.

Specifically, in step 01, cleaning may be immersion cleaning, ultrasonic cleaning, plasma cleaning, or the like, with the purpose of removing impurities on the first sheet material 10 and the second sheet material 20, to provide a clean surface for subsequent surface treatment.

In step S02, since the second sheet material 20 and the first sheet material 10 of the chip 100 are core components of the entire chip 100, the main functions of the second sheet material 20 and the first sheet material 10 are to capture DNA by means of specific surface properties, then cause the DNA to react with a biochemical reaction sequencing reagent in the chip 100, an instrument ultimately detecting a signal on the second sheet material 20 or the first sheet material 10. The performance of the second sheet material 20 and the first sheet material 10 directly affects the biochemical reaction effect and test results. Therefore, the surface treatment of the second sheet material 20 and the first sheet material 10 is a very important link.

Specifically, the surface treatment of the second sheet material 20 and the first sheet material 10 may be a chemical vapor deposition method, and this treatment method can ensure that desired functional groups are formed in a uniform distribution on the surfaces of the second sheet material 20 and the first sheet material 10 of the chip 100. The effect of surface treatment is to form a hydrophilic or hydrophobic surface according to specific needs, or to form a specific functional group, which may be a hydroxyl group, a carboxyl group, an ether bond, an aldehyde group, a carbonyl group, etc., all of which are intended to effectively connect with and affix the DNA while causing the biochemical reaction sequencing reagent to more effectively react inside the flow lane. The temperature of the surface treatment ranges from 25°C to 50°C, and the time of the surface treatment ranges from 5 min to 60 min.

The surface treatment method of the second sheet material 20 and the first sheet material 10 may also be a liquid phase immersion method, a surface spin coating method, a surface spray coating method, etc.

In step S03, the moisture on the first sheet material 10 and the second sheet material 20 may be removed by using nitrogen gas to reduce the oxidation of the first sheet material 10 and the second sheet material 20.

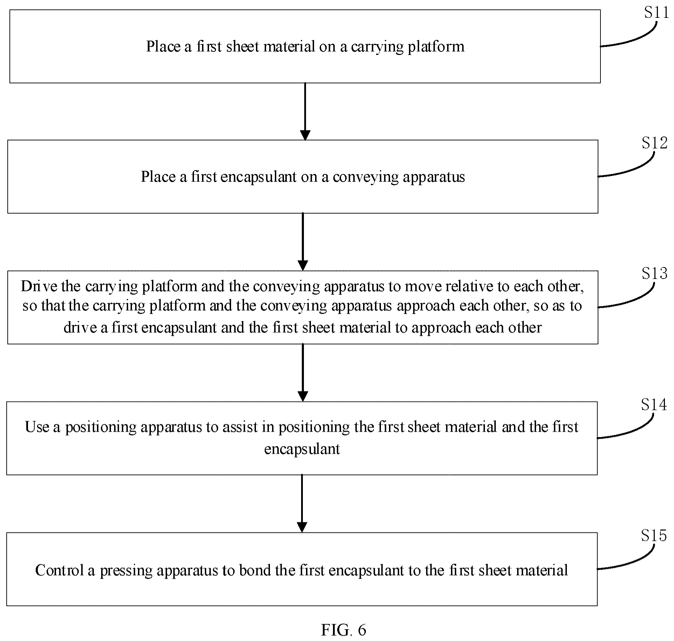

With reference to FIG. 6, in some embodiments, disposing the first encapsulant 30 on the first sheet material 10 (step S10) comprises:
S11. Placing the first sheet material 10 on a carrying platform;
S12. Placing the first encapsulant 30 on a conveying apparatus;
S13. Driving the carrying platform and the conveying apparatus to move relative to each other, so that the carrying platform and the conveying apparatus approach each other, so as to drive the first encapsulant 30 and the first sheet material 10 to approach each other;
S14. Using a positioning apparatus to assist in positioning the first sheet material 10 and the first encapsulant 30; and
S15. Controlling a pressing apparatus to bond the first encapsulant 30 to the first sheet material 10.

In this way, by means of driving the conveying apparatus and the carrying platform to move relative to each other, and using the positioning apparatus to assist in accurate positioning of the first sheet material 10 and the first encapsulant 30, the bonding accuracy of the first encapsulant 30 and the first sheet material 10 after being pressed by the pressing apparatus is improved, thereby reducing defects of the first sheet material 10 and the first encapsulant 30.

Specifically, in step S11, the carrying platform may provide support for the first sheet material 10, the carrying platform is provided with a mounting position, and the carrying platform may form a negative pressure to suction the first sheet material 10 at the mounting position. The surface shape of the carrying platform is not strictly limited, and illustratively, the carrying platform may be generally a rectangular plate-like structure.

In step S12, the conveying apparatus is provided with a mounting position, and the conveying apparatus may form a negative pressure to suction the first encapsulant 30 at the mounting position.

In step S13, the conveying apparatus may move or rotate to convey the first encapsulant 30 from a loading position distant from the carrying platform to a bonding position close to the carrying platform. The distance between the conveying apparatus and the carrying platform may be adjusted according to actual needs.

In step S14, the positioning apparatus may comprise a camera and a display screen, the camera and the display screen may be located above the carrying platform, and the conveying apparatus may be located between the carrying platform and the positioning apparatus. Positioning may be performed by diagonally aligning the first encapsulant 30 and the first sheet material 10, so as to ensure the bonding accuracy.

In step S15, the pressing apparatus may be a roller for rolling compression, or a plate-like structure for pressing compression. The pressing apparatus may roll along the length direction of the carrying platform, or may move up and down along the height direction of the carrying platform, so that the first sheet material 10 is tightly bonded to the first encapsulant 30. During bonding, a thin film of the first encapsulant 30 may be torn off first, and then a side of the first encapsulant 30 located on the thin film bonded to the first sheet material 10.

In some embodiments, before the second encapsulant 40 is disposed on the edge of the lane 31, the method comprises:
Performing defoaming treatment on the first sheet material 10 provided with the first encapsulant 30 by using a defoaming device.

In this way, bubbles generated when the first encapsulant 30 is bonded to the first sheet material 10 may be removed, increasing the adhesion between the first encapsulant 30 and the first sheet material 10, and improving the structural stability of the chip 100.

Specifically, the defoaming treatment may be that the first sheet material 10 having the first encapsulant 30 bonded thereto is placed in a defoaming machine, and a defoaming operation is performed for 3 minutes under conditions of the temperature being 40°C and the air pressure being 0.45 MPa.

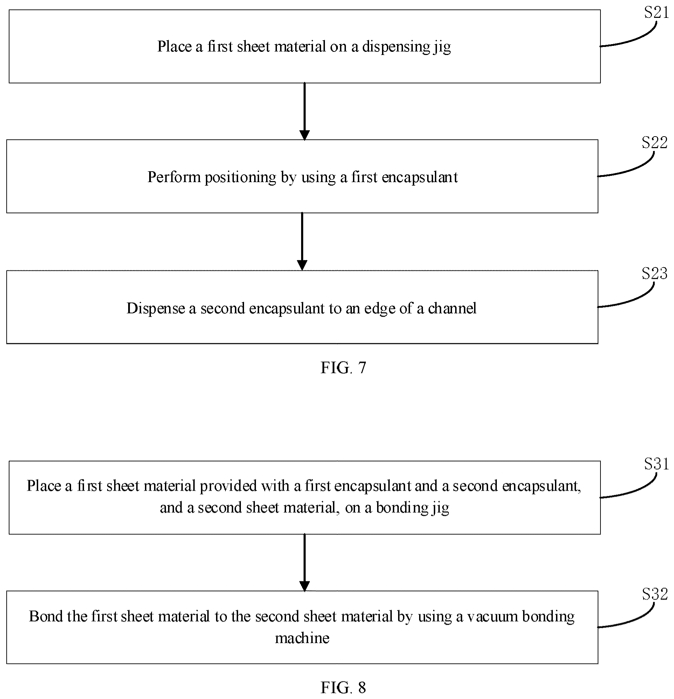

With reference to FIG. 7, in some embodiments, disposing the second encapsulant 40 at the edge of the lane 31 (step S20) comprises:
S21. Placing the first sheet material 10 on a dispensing jig;
S22. Performing positioning by using the first encapsulant 30; and
S23. Dispensing the second encapsulant 40 at the edge of the lane 31.

Specifically, in step S21, the first sheet material 10 may be placed on the dispensing jig manually, or the first sheet material 10 may be placed on the dispensing jig by a mechanical manipulator. The dispensing jig may be located on a carrying platform of the dispensing machine, a mounting space being formed on the dispensing jig, and the carrying platform of the dispensing machine may form a negative pressure to suction the first sheet material 10 in the mounting space.

In step S22, rough dispensing positioning is performed by using positioning points on the first encapsulant 30, and a certain dispensing offset is provided by using an adhesive edge for edge grabbing in the middle of each dispensing.

In step S23, the second encapsulant 40 may be dispensed at a position 0.1 mm to 0.15 mm away from the edge of the lane 31, and before dispensing, a thick film of the second encapsulant 40 may be torn off.

With reference to FIG. 8, in some embodiments, bonding the first sheet material 10 provided with the first encapsulant 30 and the second encapsulant 40 to the second sheet material 20 (step S30) comprises:
S31. Placing the first sheet material 10 provided with the first encapsulant 30 and the second encapsulant 40, and the second sheet material 20, on a bonding jig; and
S32. Bonding the first sheet material 10 to the second sheet material 20 by using a vacuum bonding machine.

Specifically, in step S31, the bonding jig may be located on a carrying platform of the vacuum bonding machine, a mounting space being formed in the bonding jig, and the first sheet material 10 being placed in the mounting space. The second sheet material 20 is located at a side of the first sheet material 10 facing away from the carrying platform. It is also possible that the second sheet material 20 is placed in the mounting space, and the first sheet material 10 is located at a side of the second sheet material 20 facing away from the carrying platform.

In step S32, the bonding principle of the vacuum bonding machine is that the first sheet material 10 and the second sheet material 20 are placed in a vacuum box in a vacuum environment, an inner mold of a vacuum cylinder is lowered by using the cylinder pressure of the machine, and a glass cover plate placed in a lower mold of the vacuum cylinder is completely pressed against a liquid crystal screen.

The degree of vacuum during bonding may be 10 Pa to 50 Pa, e.g., 10 Pa, 20 Pa, 30 Pa, 40 Pa, 50 Pa, etc. The air pressure may be 0.05 Mpa to 0.1 Mpa, e.g. 0.05 Mpa, 0.06 Mpa, 0.07 Mpa, 0.08 Mpa, 0.09 Mpa, 0.1 Mpa, etc. The bonding time may be 10 s to 30 s, e.g., 10 s, 15 s, 20 s, 25 s, or 30 s. The parameters can be set according to different vacuum bonding machines, and the bonding pressure may be about 50 N, e.g. 45 N, 47 N, 49 N, 51 N, 53 N, 55 N, etc.

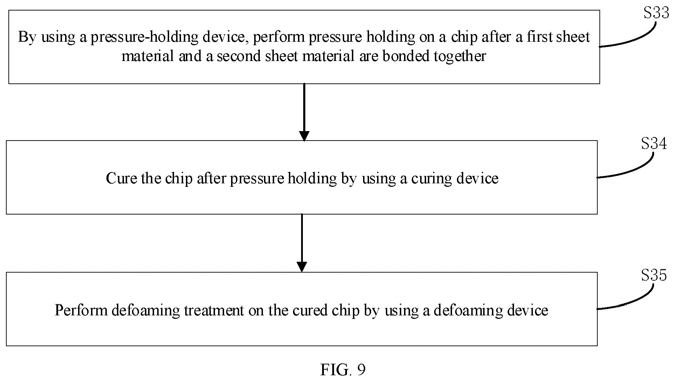

With reference to FIG. 9, in some embodiments, after the first sheet material 10 provided with the first encapsulant 30 and the second encapsulant 40 is bonded to the second sheet material 20, the method comprises:
S33. Performing pressure holding on the chip after the first sheet material 10 and the second sheet material 20 are bonded together, by using a pressure-holding device;
S34. Curing the chip 100 after pressure holding by using a curing device; and
S35. Performing defoaming treatment on the cured chip 100 by using a defoaming device.

Specifically, in step S33, the air pressure and time for pressure holding may be determined according to an actual pressure holding effect, so that the second encapsulant 40 will not burst due to excessive impact force generated on the second encapsulant 40 in the pressure holding process. Meanwhile, the chip 100 is pressed again with a certain pressure holding force, to remove some large bubbles formed when the first sheet material 10 and the second sheet material 20 are bonded together, or to change large bubbles into small bubbles. In one example, the air pressure for pressure holding is 0.3 Mpa, and the time for pressure holding is 30 s.

In step S34, the curing time and the curing power may be determined according to the curing energy of the second encapsulant 40 and the power of the curing device. In one example, the curing time is 20 s, the curing power is 90%, and the energy of the curing device is greater than 7000 mj/cm².

In step S35, the cured chip 100 is defoamed to remove small bubbles after pressure holding and curing. In one example, the defoaming time is 15 minutes, and the air pressure for defoaming is 0.45 MPa.

In some embodiments, curing the chip 100 after pressure holding by using the curing device comprises:
Emitting ultraviolet light to the second encapsulant 40 by using the curing device, so as to cure the second encapsulant 40.

Specifically, the second encapsulant 40 may be a UV adhesive, which is also referred to as an ultraviolet curing adhesive. UV adhesives are a type of adhesive that can be cured only by ultraviolet light irradiation, and may be used as a binder, or may be used as a glue material for paint, coating, ink, etc. The curing principle of UV adhesives is that a photoinitiator (or a photosensitizer) in a UV curing material absorbs ultraviolet light under the irradiation of ultraviolet light, to generate active free radicals or cations, which initiates monomer polymerization and crosslinking chemical reactions, causing the adhesive to convert from a liquid state to a solid state within a few seconds. The stronger the ultraviolet light, the faster the curing speed.

In the description of the present specification, description with reference to terms such as "one embodiment", "some embodiments", "exemplary embodiments", "example", "specific example", or "some examples" indicates that a specific feature, structure, material or characteristic described in conjunction with the described embodiment or example is included in at least one embodiment or example of the present invention. In the present specification, the schematic expressions of the described terms do not necessarily refer to the same embodiment or example. Moreover, the specific feature, structure, material or characteristic described may be combined in any one or more embodiments or examples in a suitable manner.

Although the embodiments of the present invention have been illustrated and described, it may be appreciated by those of ordinary skill in the art that changes, modifications, substitutions and variations can be made to these embodiments without departing from the principles and spirit of the present invention, the scope of which is defined by the appended claims and their equivalents.

## Claims

1. A chip, **characterized by** comprising:
a first sheet material;
a second sheet material, the second sheet material being disposed opposite the first sheet material;
a first encapsulant, the first encapsulant being disposed between the first sheet material and the second sheet material, and a lane being hollowed out in the first encapsulant; and
a second encapsulant, the second encapsulant being disposed around a side wall edge of the lane.

2. The chip according to claim 1, wherein an outer edge of the first encapsulant is flush with outer edges of the first sheet material and the second sheet material.

3. The chip according to claim 1, wherein the second encapsulant defines the edge of the lane.

4. The chip according to claim 1, wherein the first encapsulant and the second encapsulant do not overlap in a thickness direction of the first sheet material.

5. The chip according to claim 1, wherein the second encapsulant connects the first sheet material to the second sheet material.

6. The chip according to claim 1, wherein the first encapsulant is a pressure-sensitive adhesive.

7. The chip according to claim 1, wherein the second encapsulant is a UV adhesive.

8. The chip according to claim 1, wherein a thickness of the first sheet material is less than a thickness of the second sheet material.

9. The chip according to claim 1, wherein surfaces of the first sheet material and the second sheet material facing each other are both flat surfaces;
optionally, wherein a distance between the first sheet material and the second sheet material ranges from 50 µm to 200 µm.

10. The chip according to claim 1, wherein a width of the second encapsulant ranges from 0.2 mm to 0.3 mm.

11. The chip according to claim 1, wherein a width of the lane ranges from 3 mm to 15 mm, and/or a length of the lane ranges from 50 mm to 200 mm;
optionally, wherein end portions of the lane in a length direction form a converging shape;
optionally, wherein a minimum distance between two adjacent lanes is 2.0 mm.

12. An encapsulation method for a chip, **characterized by** comprising:
disposing a first encapsulant on a first sheet material, a lane being formed in the first encapsulant;
disposing a second encapsulant on an edge of the lane; and
bonding the first sheet material provided with the first encapsulant and the second encapsulant to a second sheet material.

13. The encapsulation method according to claim 12, wherein before disposing the first encapsulant on the first sheet material, the method comprises:
cleaning the first sheet material and the second sheet material;
performing surface treatment on the cleaned first sheet material and second sheet material; and
drying the surface-treated first sheet material and second sheet material;
optionally, wherein disposing the first encapsulant on the first sheet material comprises:
placing the first sheet material on a carrying platform;
placing the first encapsulant on a conveying apparatus;
driving the carrying platform and the conveying apparatus to move relative to each other, so that the carrying platform and the conveying apparatus approach each other, so as to drive the first encapsulant and the first sheet material to approach each other;
using a positioning apparatus to assist in positioning the first sheet material and the first encapsulant; and
controlling a pressing apparatus to bond the first encapsulant to the first sheet material.

14. The encapsulation method according to claim 12, wherein before disposing the second encapsulant on the edge of the lane, the method comprises:
performing defoaming treatment on the first sheet material provided with the first encapsulant by using a defoaming device. optionally, wherein disposing the second encapsulant on the edge of the lane comprises:
placing the first sheet material on a dispensing jig;
performing positioning by using the first encapsulant; and
dispensing the second encapsulant to the edge of the lane.

15. The encapsulation method according to claim 12, wherein bonding the first sheet material provided with the first encapsulant and the second encapsulant to the second sheet material comprises:
placing the first sheet material provided with the first encapsulant and the second encapsulant, and the second sheet material, on a bonding jig; and
bonding the first sheet material to the second sheet material by using a vacuum bonding machine;
optionally, wherein after the first sheet material provided with the first encapsulant and the second encapsulant is bonded to the second sheet material, the method comprises:
by using a pressure-holding device, performing pressure holding on the chip after the first sheet material and the second sheet material are bonded together;
curing the chip after pressure holding by using a curing device; and
performing defoaming treatment on the cured chip by using a defoaming device;
optionally, wherein curing the chip after pressure holding by using the curing device comprises:
emitting ultraviolet light to the second encapsulant by using the curing device, so as to cure the second encapsulant.
